(19) Europäisches Patentamt
European Patent Office
Office européen des brevets

(11) Publication number: **0 035 860**
**B1**

(12) **EUROPEAN PATENT SPECIFICATION**

(45) Date of publication of patent specification: **14.03.84**

(21) Application number: **81300873.7**

(22) Date of filing: **03.03.81**

(51) Int. Cl.³: **C 07 C 69/16,**
C 07 C 67/29,
C 07 C 67/37,
C 07 C 47/06, C 07 C 45/49
//B01J23/44, B01J27/08

(54) **Process for producing ethylidene diacetate and/or acetaldehyde.**

(30) Priority: **06.03.80 JP 28433/80**

(43) Date of publication of application:
**16.09.81 Bulletin 81/37**

(45) Publication of the grant of the patent:
**14.03.84 Bulletin 84/11**

(84) Designated Contracting States:
**DE FR GB IT**

(56) References cited:
**FR - A - 2 303 788**

(73) Proprietor: **MITSUBISHI GAS CHEMICAL COMPANY, INC.**
**5-2, Marunouchi 2-chome Chiyoda-Ku**
**Tokyo (JP)**

(72) Inventor: **Isshiki, Tomiya**
**14-11, Umegaoka 2-chome Setagaya-ku**
**Tokyo (JP)**
Inventor: **Ito, Akira**
**15-6, Nishikubo-cho Tokiwadaira**
**Matsudo-shi Chiba-ken (JP)**
Inventor: **Kijima, yasuhiko**
**641, Koda**
**Matsudo-shi Chiba-ken (JP)**
Inventor: **Watanabe, Takayuki**
**6-1 Kanamachi 1-chome**
**Katsushika-ku Tokyo (JP)**

(74) Representative: **Ritter, Stephen David et al,**
**Mathys & Squire 10 Fleet Street**
**London EC4Y 1AY (GB)**

**EP 0 035 860 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

# Process for producing ethylidene diacetate and/or acetaldehyde

This invention relates to a process for producing ethylidene diacetate and/or acetaldehyde which comprises causing hdyrocarbonylation reaction of methyl acetate or dimethyl ether with carbon monoxide and hydrogen in the presence of a catalyst comprising as a main component palladium supported on a porous inorganic material and as a secondary component at least one halide selected from the group consisting of iodide, bromide and mxitures thereof under substantially anhydrous conditions.

In the prior art, ethylidene diacetate was synthesized from acetylene and acetic acid or by reacting acetaldehyde with acetic anhydride.

Recently, a process for producing ethylidene diacetate by reacting methyl acetate or dimethyl ether with carbon monoxide and hydrogen in the presence of a catalyst was proposed (refer to Japanese Patent Publication (laid open) No. 115409/1976). In the process disclosed in this Patent Publication, noble metals belonging to Group VIII of the Periodic Table and halides were essential components of the catalyst for hydrocarbonylation reaction. There is the passage "co-catalysts can be used together with the noble metal catalyst in order to enhance the reactivity" in the Patent Publication. In Working Examples 1—29 of the Patent Publication, rhodium salt or complex, or palladium salt or complex was used as the main catalyst. Besides, in Working Examples 5 and 8, little ethylidene diacetate is formed in the absence of an organic nitrogen group compound or an inorganic metal compound. In other words, the invention of Patent Publication No. 115409/1976 relates primarily to a process for synthesizing ethylidene diacetate from methyl acetate or dimethyl ether, carbon monoxide and hydrogen in the presence of a catalyst consisting of (i) a metal compound, which is soluble in the reaction mixture, selected from the group consisting of rhodium salt or complex and palladium salt or complex and (ii) a halide and (iii) a co-catalyst composed of an organic nitrogen group compound or an inorganic metal compound. In the process disclosed in the patent publication a liquid-phase homogeneous reaction is effected by using a rhodium salt or complex, or a palladium salt or complex. Patent Publication No. 115409/1976 also suggests the use as catalyst of a Group VIII noble metal in the zero valent state and in finely divided form but gives no working examples to support this suggestion. (In fact it has been found by the present applicants, see Control Test 1 below, that palladium in the zero valent state cannot catalyse the carbonylation of methyl acetate with carbon-monoxide and hydrogen). Patent Publication No. 115409/1976 also suggests the use of noble metal catalysts which are chemically bonded to a polymeric substrate. It further states that although these forms of catalyst are heterogeneous in the physical sense, these catalysts are nevertheless to be regarded as special forms since they display characteristics which are more akin to homogeneous than to heterogeneous catalysts. Furthermore they are prepared in a special way by chemically bonding the Group VIII noble metal to the substrate through an organic promotor.

When a noble metal, such as rhodium and palladium is used as a homogeneous catalyst, expensive rhodium and palladium must be recovered from the homogeneous reaction mixture after completing the reaction. However, in general, such recovery needs complicated steps and loss of the noble metal cannot be avoided in the recovering step. This loss is described in Hydrocarbon Processing 54 June 83 (1975), Patent Publication (laid open) No. 90204/1978 and JAPAN CHEMICAL INDUSTRY ASSOCIATION MONTHLY, 14th March 1973. As a result, the resulting product becomes costly.

In order to simplify the separation of a product from a reaction mixture and recovery of a catalyst employed and in order to make easy circulation of a reaction solution in a continuous reaction a heterogeneous catalyst is effective, so development of such catalyst is very important. (Refer to CHEMTECH 560, 1973 by Charles U. Pihman, Jr. and George O. Evans).

Referring Z. M. Michalska and D. E. Webster: "Supported Homogeneous Catalyst" CHEMTECH, 117 (1975), Patent Publication (laid open) No. 115409/1976 suggests disclose an organic polymer metal complex containing a main chain composed of polystyrene-divinylbenzene as a heterogeneous catalyst.

Many solid catalysts containing polymer-metal complex, namely ion-exchange resin as a main component, are used on a laboratory scale. However, the catalysts must be used at a temperature less than 100°C. (Organo Inc. catalog and Mitsubishi Chemical Industries Inc. Catalog).

At a temperature more than 100°C, physical durability and chemical stability of the catalyst are poor, and reaction rate and selectivity in case of using such heterogeneous catalyst are inferior to those in case of using a homogeneous catalyst; metal in the catalyst is released from the catalyst and is dissolved in the liquid phase; and the catalyst tends to be deactivated. As explained above the known heterogeneous prior catalyst have fatal shortcomings. So, it is difficult to use such catalysts on an industrial scale.

The present inventors carried out research to find a process for synthesizing ethylidene diacetate using a heterogeneous catalyst.

As a result, we found that palladium physically supported on porous inorganic materials is effective for synthesis of ethylidene diacetate.

An object of this invention is to provide a process for synthesis of ethylidene diacetate and/or

acetaldehyde in which recovery of the catalyst employed is easy.

This invention thus relates to a process for producing ethylidene diacetate and/or acetaldehyde which comprises reacting methyl acetate or dimethyl ether, carbon monoxide and hydrogen in the presence of a catalyst consisting essentially of (i) as a main component a group VIII noble metal and (II) as a secondary component at least one halide selected from iodides, bromides and mixtures thereof under substantially anhydrous conditions. The process of the invention is characterised in that the main catalyst component comprises palladium physically supported on a porous inorganic material having a pore volume of 0.03 to 2.5 $cm^3/g$ and a surface area of 0.1 to 1500 $m^2/g$.

Though expensive rhodium (belonging to Group VIII of the Periodic Table) supported on a porous inorganic materials have effective catalytic activity, rhodium supported on the material tends to be released from the material and be dissolved in the liquid phase. Though ruthenium is cheaper than rhodium, ruthenium supported on a porous inorganic materials are poor catalytic activity. Similarly, platinum supported on a porous inorganic materials are poor in catalytic reactivity. Refer to Control tests 1 and 2 as explained below. On the other hand, we found that palladium physically supported on a porous inorganic materials are effective for such synthesis of ethylidene diacetate and/or acetaldehyde, because they have excellent catalytic activity and little palladium is released from the material. As shown in the foregoing Examples, more than 90.0%, in general more than 99.0% of the palladium remains intact on the porous inorganic material per hour; and more than 99.9% of the palladium remains intact on the porous inorganic material under preferable conditions per hour.

A catalyst consisting of metallic palladium, a halide and a promoter did not have catalytic activity. (refer to control test 1). In other words, a catalyst comprising palladium supported on a porous inorganic material and a bromide or an iodide not only has high catalytic activity but also prevents the palladium from dissolving in the reaction solution.

It could not be anticipated from the prior art that a palladium supported on a porous inorganic material has the above mentioned advantages. This is proved by comparing reaction speed, yield and resistance to palladium's dissolving into reaction system of this invention with those of the prior art systems disclosed in [K. M. Webber and B. C. Cates, J. Mol. Catal. 3(1977/78)1—9].

The mechanism of the hydrocarbonylation reaction of methyl acetate or dimethyl ether is not perfectly clear. However, it is believed that the reaction by which ethylidene diacetate is synthesized can be expressed in the following equations:

(1) When methyl acetate is used as a raw material

$$2CH_3COOH_3 + 2CO + H_2 \rightarrow CH_3CH(OCOCH_3)_2 + CH_3COOH$$

(2) When dimethyl ether is used as a raw material

$$2CH_3OCH_3 + 4CO + H_2 \rightarrow CH_3CH(OCOCH_3)_2 + CH_3COOH$$

The main component employed in the practice of this invention may be prepared by supporting palladium on a porous inorganic material by coprecipitation, impregnation, blending, adsorption, ion exchange and the like. One of the processes for supporting palladium on a porous inorganic material is as follows: A porous inorganic material is impregnated with a solution of palladium metal or a palladium compound and other components, followed by reducing the palladium compound in the material to palladium metal with a reducing agent, such as formalin, hydrogen, sodium formate, carbon monoxide, sodium borohydride, lithium aluminum hydride, or hydrazine and then drying the resulting palladium supported on the material. Of course, the palladium supported on the material may be prepared by other methods.

The palladium to be supported on a porous material can be used in any form of zero valence state and higher valence state. Palladium metal or palladium compounds include for example metallic palladium, PdO, $Pd(OH)_2$, $Pd(NO_3)_2$, $Pd(NO_2)_2$, $PdSO_4$, $Pd(PO_4)_2$, $[Pd(NH_3)_4]X_2$, $(NH_4)_2PdX_4$, $Na_2PdX_4$, $K_2PdX_4$, $Li_2PdX_4$, $PdX_2$, $Pd(OAc)_2$, $Pd(AcAc)_2$, and $PdX_2(PhCN)_2$ wherein X is Cl, Br or I, Ph is phenyl group, OAc is acetoxy group and AcAc is acetylacetonate group.

The porous inorganic materials employed for supporting palladium are the ones having particle size of 400 mesh to 1 inch so that palladium supported on a porous inorganic materials may be used in a fluidized bed reactor or fixed bed reactor. The pore volume of the material in proportion to weight of the material may be in the range of 0.03 to 2.5 $cm^3/g$, preferably 0.05 to 1.5 $cm^3/g$. Surface area of the material in proportion to weight of the material may be in the range of 0.1 to 1500 $m^2/g$, preferably 1 to 1500 $m^2/g$. Average semi-diameter of the pore in the material may be in the range of 0.1 to 20000 Å, preferably 1 to 2000 Å and most preferably 5 to 1500 Å.

The ratio of palladium to a porous inorganic material is not critical and any amount of palladium can be supported on a porous inorganic material. In general, the proportion by weight of palladium to be supported on a porous inorganic material may be in the range of 0.0001 to 50%, preferably 0.001 to 40%, more preferably 0.01 to 30% and most preferably 0.05 to 20%.

Examples of the porous inorganic material include carbon, activated carbon, graphite, bone black, alumina, silica, silica alumina, barium sulfate, natural or synthetic zeolite, spinel, magnesia adhered

alumina, thoria, titanium oxide, zirconium oxide, thorium oxide, lanthanum oxide, cerium oxide, zinc oxide, tantalum, clay, diatomaceous earth, Celite (trade name of product being commercially available from Johns-Manville Co.), asbestos, pumice stone, bauxite, terra alba, natural and treated terra alba, such as Super-Filtrol, silicon carbide, zeolite molecular sieves, ceramic honeycomb, boria, cements, alundum, corundum, brick, talc, gypsum and the like. Carbon, graphite, bone black, alumina, silica, silica alumina, barium sulfate, zeolite spinel and magnesia adhered alumina are preferable. Activated carbon, graphite, bone black, alumina, silica, silica alumina and barium sulfate are more preferable. Activated carbon is most preferable. The carriers may be in the form of particles having uniform or non-uniform size. The carriers may be in the form of molded articles, extruded articles, ceramic bars, balls, fragments, tiles and the like. The palladium supported on a porous inorganic materials can easily be prepared from commercially available compounds. Special conditions are not required for the preparation of the palladium supported on a porous inorganic materials. The palladium can be released from the material to regenerate the main component.

An iodide, a bromide and mixture thereof may be used as a secondary component. An iodide is preferred.

Examples of the halides include methyl halides, acetyl halides, hydrogen halides and mixtures thereof. Methyl iodide, acetyl iodide hydrogen iodide or mixture thereof can be used as a halide. An iodide and/or a bromide may be added to the reaction system as it is.

Materials which can convert to methyl halides, such as methyl iodide; acetyl halide, such as acetyl iodide or hydrogen halides, such as hydrogen iodide by reacting with components in the reaction system can also be used as a secondary component. Examples of the materials include inorganic halides, such as alkali metal halides, such as lithium halides, sodium halides, or potassium halides; alkaline earth metal halides, such calcium halides or magnesium halides; metal halides, such as aluminum halides, zinc halides, copper halides, lanthanum halides, or cerium halides; and halogens, such as bromine or iodine.

The hydrocarbonylation reaction of methyl acetate or dimethyl ether of this invention sufficiently proceeds in the presence of the catalyst containing the above mentioned main component and the above mentioned secondary component. Promoters composed of compounds of nitrogen family elements may be used with the catalyst in order to increase the reaction rate. Preferable compounds of nitrogen family elements include organic compounds containing nitrogen, phosphorus, antimony or arsenic, ammonia or ammonia salts.

Examples of the compounds are shown in the following. However, compounds which are not listed in the following may be used as a promoter.

(I) trivalent compounds of nitrogen family elements

(A) Compounds represented by the formula

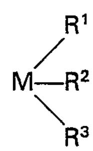

wherein M is N, P, Sb or As.

(a) compounds wherein $R^1$, $R^2$ and $R^3$ may be the same or different, and are independently hydrogen, saturated alkyl having 1—10 carbon atoms, saturated cycloalkyl having 3—10 carbon atoms, or aryl

M is N

The compounds include, for example, ammonia, and amines, such as monomethyl amine, dimethyl amine, trimethyl amine, monoethylamine, diethyl amine, triethyl amine, dimethyl ethyl amine, tri-n-propyl amine, tri-isopropyl amine, tri-n-butyl amine, tri-tert.-butyl amine, aniline, dimethyl aniline, diethyl aniline, dimethylbenzyl amine, toluidine, triphenyl amine, cyclohexyl amine and the like.

M is P

The compounds include, for example, phosphines, such as tri-n-propyl phosphine, tri-iso-propyl phosphine, tri-n-butyl phosphine, tri-tert.-butyl phosphine, tricyclohexyl phosphine, ethylene bis(diphenyl phosphine), triphenyl phosphine and the like

M is Sb

The compounds include, for example, stibines, such as tri-iso-propyl stibine, ethyl-di-iso-propyl stibine, triphenyl stibine, tri(o-tolyl)stibine, phenyl diamyl stibine, tris(diethyl aminomethyl)stibine, bis(diethylstibino)pentane and the like.

M is As

The compounds include, for example, arsines, such as trimethyl arsine, triethyl arsine, tri-iso-propyl arsine, tripropyl arsine, tricyclohexyl arsine, phenyl di-iso-propyl arsine, diphenyl arsine, bis(diphenyl arsino)ethane, bis(di-iso-propyl arsino)hexane, and the like.

(b) wherein $R^1$ is hydrogen, alkyl having 1—10 carbon atoms, cycloalkyl having 3—10 carbon atoms, or aryl and $R^2$ and $R^3$ are taken together and represents methylene or polymethylene having 2—5 carbon atoms; the compounds include, for example, pyrrolidine, N-methyl pyrrolidine or piperidine or N-phenyl piperidine:

(c) wherein $R^1$ and $R^2$ may be the same or different and independently represent hydrogen, alkyl having 1—10 carbon atoms, cycloalkyl having 3—10 carbon atoms, or aryl and $R^3$ is aliphatic saturated acyl, or $R^1$ is hydrogen, alkyl having 1—10 carbon atoms, cycloalkyl having 3—10 carbon atoms or aryl, and $R^2$ and $R^3$ are taken together and represent lactam ring; the compounds include, for example carboxylamides such as acetamide, N,N-dimethylacetamide, acetoanilide, N-methyl-N-phenyl-acetamide, and lactam, such as N-methylpyrrolidinone:

(d) wherein at least one of $R^1$, $R^2$ and $R^3$ is carboxymethyl and the remainder is hydrogen, alkyl having 1—10 carbon atoms, cycloalkyl having 3—10 carbon atoms or aryl; the compounds include, for example carboxylic acid derivatives, such as N,N-dimethyl glycine, N,N-diethyl glycine, imino diacetic acid, N-methyl iminodiacetic acid, or nitrilotriacetic acid:

(B) Organic compounds represented by the formula

$$N{\equiv}C{-}R$$

wherein R represents an alkyl having 1 to 10 carbon atoms, a cycloalkyl having 3—10 carbon atoms or aryl; the compounds include for example nitriles, such as acetonitrile, propionitrile, or benzonitrile.

(C) Organic nitrogen group compounds represented by the formula

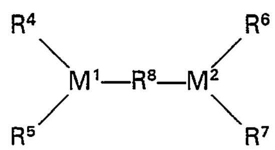

(a) wherein $R^4$, $R^5$, $R^6$ and $R^7$ may be the same or different and independently hydrogen, alkyl having 1—10 carbon atoms, cycloalkyl having 3—10 carbon atoms, or aryl and $R^8$ is methylene group or polymethylene group having 2—10 carbon atoms, phenylene, or carbonyl group

$M^1$ and $M^2$ are N, P, Sb or As

The compounds include, for example, ethylene bis(diphenylphosphine), phenylene bis(dimethyl-phosphine), bis(diphenylarsine)ethane, bis(di-iso-propylarsino)hexane, bis(diethylstibino)pentane, N,N,N',N'-tetramethylethylenediamine, N,N,N',N'-tetraethylenediamine, N,N,N',N'-tetra-n-propyl-ethylenediamine, N,N,N',N'-tetramethylmethylenediamine, N,N,N',N'-tetramethyl urea, N-methyl-2-pyrrodinone and triethylenediamine,

(b) $R^4$ and $R^6$ may be the same or different and independently represent hydrogen, alkyl having 1—10 carbon atoms, cycloalkyl having 3—10 carbon atoms or aryl and $R^5$ an $R^7$ are taken together and represent methylene or polymethylene having 2—5 carbon atoms and $R^8$ is methylene or poly-methylene having 2—5 carbon atoms; the comopunds include, for example, heterocyclic compounds, such as piperazine, N-methyl-piperazine, N-ethyl-piperazine, or 2-methyl-N,N'-dimethylpiperazine.

(c) other compounds include, for example, tris(diethylaminomethyl)stibine, 2,5-dicarboxy-piperazine, cyclohexane-1,2-diamine-N,N,N',N'-tetraacetic acid or salts thereof, tetramethylester, ethylenediaminetetraacetic acid, or its salt or tetramethylester thereof, 1,4-azabicyclo[2,2,2]octane, methyl substituted, 1,4-diazabicyclo[2,2,2]octane, adiponitrile or N-methyl-morpholine.

(II) Hetero cyclic compounds include, for example, pyridines, such as pyridine; $\alpha$-picoline, $\beta$-picoline, $\gamma$-picoline, 2-ethylpyridine, 3-ethylpyridine, 4-ethylpyridine, 2-propylpyridine, 4-propylpyridine, 4-butylpyridine, 4-isobutylpyridine, 4-tert.-butylpyridine, 2,6-lutidine, 2,4-lutidine, 2,5-lutidine, 3,4-lutidine, 3,5-lutidine, 2,4,6-collidine, 2-methyl-4-ethylpyridine, 2-methyl-5-ethylpyridine, 3-methyl-4-ethylpyridine, 3-ethyl-4-methylpyridine, 3,4-diethylpyridine, 3,5-diethylpyridine, 2-methyl-5-butylpyridine, 4-pentylpyridine, 4-(5-nonyl)-pyridine, 2,6-dipropylpyridine, 2-methyl-3-ethyl-6-propylpyridine, 2,6-diethylpyridine, 2,6-dipropylpyridine, 2,6-dibutylpyridine, 2,6-di-tert.-butylpyridine, functional group-containing pyridines, such as 2-cyanopyridine, 3-cyanopyridine, 4-cyanopyridine, 2,6-dicyanopyridine, 3,5-dicyanopyridine, 2-cyano-6-methylpyridine, 3-cyano-5-methylpyridine, picolinic amide, nicotinic amide, isonicotinic amide picolinic acid, nicotinic acid, isonicotinic acid, dipicolinic acid,

dinicotinic acid, cinchomethorinic acid, 5-butylpicolinic acid, alkyl ester of nicotinic acid, 2-aminopyridine, 3-aminopyridine, 4-aminopyridine, 2,3-diaminopyridine, 2,5-diaminopyridine, 2,6-diaminopyridine, 2,3,6-triaminopyridine, 2-amino-3-methylpyridine, 2-amino-4-methylpyridine, 2-amino-5-methylpyridine, 2-amino-6-methylpyridine, 2-amino-4-ethylpyridine, 2-amino-4-propylpyridine, 2-amino-4-(5-nonyl) pyridine, 2-amino-4,6-dimethylpyridine, 2,6-diamino-4-methylpyridine, 2,2'-dipyridylamine, 4-dimethylaminopyridine, 2-hydroxypyridine, 3-hydroxypyridine, 4-hydroxypyridine, 2,6-dihydroxypyridine, 3-hydroxy-6-methylpyridine, 2-chloropyridine, 2,6-dichloropyridine, 4-chloropyridine, 2-amino-5-chloropyridine, 2-amino-3,5-dichloropyridine, 2,6-dimethyl-3,5-dichloropyridine, 2-amino-5-chloro-3-methylpyridine, 2-amino-3,5-dichloro-4-methylpyridine, 2-amino-3,5-dichloro-4,6-dimethylpyridine, 4-amino-3,5-dichloropyridine, pyridine-N-oxide, $\beta$-picoline-N-oxide, $\alpha$-picoline-N-oxide, $\gamma$-picoline-N-oxide, 2,6-lutidine-N-oxide, 3,5-lutidine-N-oxide, 4-phenylpropylpyridine-N-oxide, 1,3-di-(4-pyridyl)-propane-di-N-oxide, 4-(5-nonyl)-pyridine-N-oxide, 2-chloropyridine-N-oxide, 4-cyanopyridine-N-oxide, 2-pyridine-methanol, 3-pyridinemethanol, 4-pyridinemethanol, 2,6-pyridine-methanol, 2-pyridineethanol, 4-pyridineethanol, 3-picolylamine, 4-picolylamine, 2-methylaminoethylpyridine, 4-alkylaminoethylpyridines, 4-piperidinoethylpyridine, 4-(4-pipecolinoethyl)pyridine, and 4-morpholinoethylpyridine, pyridines containing heterocyclic or homocyclic group such as 2-phenylpyridine, 4-phenylpyridine, 2-benzylpyridine, 4-benzylpyridine, 4-phenylpropylpyridine, 4,4'-dipyridyl, 4,4'-dimethyl-2,2'-dipyridyl, 1,3-di-(4-pyridyl)propane, 1,2-di-(4-pyridyl)-ethane, 1,2-di(4-pyridyl)-ethylene, 1,2,3-tri-(4-pyridyl)propane, 2,4,6-tri-(4-pyridyl)-S-triazine, 2,4-di-(4-pyridyl)-6-methyl-S-triazine, and 2,5-di-(4-pyridyl)-S-tetrazine, alkenylpyridine or polymer pyridine, such as 2-vinylpyridine, 4-vinylpyridine, 2-vinyl-6-methylpyridine, 2-vinyl-5-ethylpyridine, 4-butenyl pyridine, 4-vinylpyridine homopolymer, 2-vinylpyridine homopolymer, 2-vinylpyridine-acrylonitrile copolymer, 4-vinylpyridine-acrylonitrile copolymer, 4-vinylpyridine-styrene copolymer, 4-vinylpyridine-divinylbenzene copolymer, 2-vinylpyridine-divinylbenzene copolymer, and 4-vinylpyridine homopolymer-N-oxide; pyrroles; pyrrolines; pyrinidines; pyrazines; pyrazoles; pyrazolines; pyridazines; imidazoles; 1,10-phenanthrolines, such as 4-chloro-1,10-phenanthroline, and 5-(thiabentyl)-1,10-phenanthroline; quinolines, such as 2-(dimethylamino)-6-methoxyquinoline, 8-hydroxyquinoline and 2-carboxyquinoline.

(III) Pentavalent nitrogen group compounds

The compounds include, for example ammonium acetate, ammonium propionate, and triphenylphosphineiminiumchloride.

Of these nitrogen group compounds, ammonia, ammonium salts and organic compounds containing nitrogen atom or phosphorous atom are preferable; organic compounds containing trivalent phosphorus atom or trivalent nitrogen atom are more preferable; and phosphine compounds containing trivalent phosphorus atom and heterocyclic compounds containing trivalent nitrogen atom, such as pyridines are most preferable.

These promoters may be supported on a porous inorganic material with a palladium metal. Alternatively, promoters may be used without being supported on a porous inorganic material. It is preferred that promoter be supported on a porous inorganic material with a palladium metal.

The amount of the main component employed depends on type of reaction, that is, whether it is in a fixed bed or a fluidized bed.

In general, the amount of the main component employed may be in the range of from $1 \times 10^{-4}$ to 25 wt%, preferably in the range of from $1 \times 10^{-3}$ to 20 wt% more preferably from $2.5 \times 10^{-3}$ to 15 wt% and most preferably from $5 \times 10^{-3}$ to 10 wt% on the basis of weight of a reaction solution in terms of metal.

The amount of halides employed as a secondary component employed may be in the range of from $1 \times 10^{-6}$ to 15 mol, preferably $1 \times 10^{-5}$ to 5 mol, most preferably $1 \times 10^{-4}$ — 3 mol per 1 liter of a reaction solution in terms of halogen atom.

Amount of ammonia or other promoter compound employed may be in the range of from $10^{-6}$ to 10 mol, preferably $10^{-4}$ to 5 mol and more preferably $10^{-3}$ to 2.5 mol per 1 liter of a reaction solution.

In practicing this invention, the reaction temperature is not critical. In general, the reaction temperature may be in the range of 20—500°C, preferably 80—350°C, more preferably 100—250°C.

The reaction pressure is kept high enough to keep the raw material(s), the solvent and the product in a liquid phase and to maintain appropriate partial pressures of hydrogen and carbon monoxide. The partial pressure each of hydrogen and carbon monoxide may be in the range of 0.5—700 atm., preferably 1—600 atm., and more preferably 3—500 atm. However, partial pressures of hydrogen and carbon monoxide may be in the range of 0.05—1000 atm.

Dimethyl ether and methyl acetate which are used as raw materials in this invention may be prepared by known methods. For example, dimethyl ether may be produced by dehydration-dimerization reaction of methanol.

The methyl acetate may be produced by esterification of methanol and acetic acid. In this reaction acetic acid may be produced by reacting methanol with carbon monoxide (refer to Patent Publication (laid open) Nos. 59211/1979, 63002/1979 and 66614/1979). Acetic acid which is formed as a by-product in synthesis of vinyl acetate from ethylide diacetate may be used for synthesis of methyl

acetate. Synthesis of vinyl acetate from ethylidene diacetate is disclosed USP No. 2,425,389; Kogyo Kogaku Zasshi 74(9)1825(1971) and Hydrocarbon Process 44(11)287(1965). Methyl acetate may also be produced by reacting methanol with synthesis gas. [Patent Publication for opposition No. 2525/1973, Patent Publication (laid open) Nos. 136110/1977 and 136111/1977]. Vinyl acetate is converted to polyvinyl acetate. Methyl acetate formed as a by-product when the polyvinyl acetate is converted to polyvinyl alcohol may be used as a raw material. Methanol, dimethyl ether, acetic acid, acetaldehyde, dimethyl acetal and iodides, such as methyl iodide be incorporated in the methyl acetate employed as a raw material of this invention as long as an overall balance is obtained.

Preparation of methyl acetate from acetic acid formed as a by-product in synthesis of vinyl acetate from ethylidene diacetate is preferable in an overall process for producing vinyl acetate from methanol and synthesis gas.

Stoichiometric amounts of the raw material gas depend on which one of methyl acetate, dimethyl ether and mixture thereof is used. Therefore, stoichimetric amounts of carbon monoxide and hydrogen may vary from molar ratio of CO to $H_2$ of 2:1 to molar ratio of CO to $H_2$ of 4:1. It is not necessarily critical to use CO and $H_2$ in stoichiometric amounts. In general, the molar ratio of CO to $H_2$ may be in the range of from 1:100 to 100:1, preferably 1:50 to 50:1 and more preferably 1:10 to 10:1. However, it is preferable that carbon monoxide and hydrogen be used in an approximately stoichiometric amount. So, preferably, the ratio of carbon monoxide to hydrogen may be in the range of 0.5:1 to 5:1.

In the practice of this invention, carbon monoxide and hydrogen may be introduced into the reaction system as separate streams. Synthesis gas comprising carbon monoxide and hydrogen may be introduced into the reaction system. The raw material gas composed of carbon monoxide and hydrogen does not need to be highly pure and may contain carbon dioxide, methane, nitrogen, and rare gases. Extremely low concentrationn of either of carbon monoxide or hydrogen in the mixed gas is not desirable, because the reaction pressure must rise when using such gas.

In the present process, methyl acetate or dimethyl ether as a raw material and ethylidene diacetate or acetaldehyde as a product serve as a solvent for reaction, so another solvent is not necessary. An organic solvent and a diluent compatible with the raw materials and the product under the reaction conditions may be used. Examples of the organic solvent include organic acids, such as acetic acid, acetic anhydride, propionic acid, butyric acid, octanoic acid, phthalic acid, benzoic acid; esters of organic acids, such as methyl acetate, ethyl acetate, ethylene glycol diacetate, propylene glycol diacetate, dimethyl adipate, methyl benzoate, ethyl benzoate, dimethyl phthalate, diethyl phthalate, dioctyl phthalate, phenyl acetate, and tolyl acetate; hydrocarbons, such as dodecane, hexadecane, benzene, naphthalene, and biphenyl; esters of inorganic acids, such as triphenyl phosphate, tricresyl phosphate, dibutylphenyl phosphate, silicates such as tetramethyl ortho-silicate, and tetrabutyl silicate; aromatic ethers, such as diphenyl esters; ketnones, such as acetone, methyl ethyl ketone, dibutyl ketone, methyl isobutyl ketone, acetophenone, and benzophenone. The use of acetic acid or acetic anhydride as a solvent is preferred in the present invention.

The product, ethylidene diacetate and/or acetaldehyde produced according to this invention is likely to decompose in the presence of water. After the raw material and the solvent have been dried to a substantially anhydrous state, they should be introduced into the reaction system. The reaction system should be kept in a substantially anhydrous state. By substantially anhydrous conditions is meant that the water content of the reaction system is less than 10 mol% on the basis of total mol of the liquid components present in the system. In general, a water content of less than 5 mol% is preferable, and a water content of less than 3 mol% gives best results. When the materials contain a large amount of water, they should be dried before introducing them into the reaction system. Though commercially available synthesis gas and methyl acetate or dimethyl ether contain water, in general, the water content of these materials is within the above limits. So, commercially available synthesis gas and methyl acetate or dimethyl ether can be used as they are as raw materials of this invention.

The present process may be carried out by batch, semicontinuous or continuous methods. The reaction may be effected in either of a fixed bed or a fluidized bed. In continuous process, the raw materials and the catalyst are continuously introduced into the reaction zone for the hydrocarbonylation reaction is effected; and the reaction mixture is continuously removed from the zone. The ethylidene diacetate and/or acetaldehyde is separated from the reaction mixture, for example, distillation.

In another embodiment, the reaction mixture containing ethylidene diacetate and/or acetaldehyde removed from the reaction zone is introduced into another reaction zone as it is, or alternatively after part of the components other than ethylidene diacetate and/or acetaldehyde have been separated from the reaction mixture, the resulting reaction mixture may then be introduced into another reaction zone. In the reaction zone, ethylidene diacetate may be converted to acetic acid and vinyl acetate. Methyl acetate and acetic acid may be recovered from the resulting reaction mixture by distillation to leave vinyl acetate as a product. Esterification of the recovered acetic acid and methanol may be effected to form methyl acetate. The methyl acetate thus formed and unreacted methyl acetate may be introduced into the hydrocarbonylation reaction zone.

As explained above, ethylidene diacetate and/or acetaldehyde is produced by using a novel solid

catalyst according to the present invention. So, this invention is significant from an industrial point of view.

According to the present invention, ethylidene diacetate alone, acetaldehyde alone and mixture of ethylidene diacetate and acetaldehyde can be obtained through the hydrocarbonylation reaction. The mixture can be separated to each compound by known methods. The mixture can be used for other purpose without separation to each compound. It is preferable to obtain ethylidene diacetate as a main product through the hydrocarbonylation reaction.

The following examples are given as illustrative embodiments of the invention and should not be construed as limiting its scope.

All parts and percents are by weight, unless otherwise specified.

Example 1

Into a 100 ml autoclave were charged methyl acetate (20.0 g), acetic acid (15.0 g), methyl iodide (13.0 g), 2,6-lutidine (0.367 g) and palladium (5%) supported on activated carbon (1.44 g) being commercially available from Nippon Engelhard. Air in the autoclave was purged with nitrogen. The nitrogen gas was purged with mixed gas of carbon monoxide and hydrogen (ratio by volume of $CO:H_2$ or 2:1).

The temperature in the autoclave was raised to 175°C. The above mixture of CO and $H_2$ under pressure was charged into the autoclave, until the pressure in the autoclave reached to 100 $Kg/cm^2$ Gauge. The reaction was continued for 4 hours at 175°C with stirring while maintaining the pressure in the autoclave at 100 $Kg/cm^2$ Gauge by continuously introducing the mixed gas into the autoclave.

Then, the reaction mixture was cooled, and removed from the autoclave. Analysis showed that it contained 4.12 g of ethylidene diacetate, 0.0967 g of acetic anhydride and 0.88 g of acetaldehyde, and that the remaining components were mainly methyl acetate, acetic acid and methyl iodide.

The palladium supported on activated carbon was separated from the reaction mixture. Atomic-absorption spectroscopy showed that in comparison with palladium supported on the activated carbon before using, 99.98% of the palladium remained intact on the activated carbon.

Example 2

Alumina-magnesia (alumina:magnesia = 9:1), 100—200 mesh, commercially available from Mizusawa Chemical KK, was washed with an NaOH solution and water. A palladium chloride solution in hydrochloric acid was added to the alumina-magnesia so treated. The mixture was stirred at 80°C for a given time. The pH of the solution was adjusted to 9—10 by adding water and an NaOH solution thereto, and the palladium compound supported on alumina-magnesia was reduced with formalin. The pH of the solution was then adjusted to 7 by adding hydrochloric acid thereto, after which the palladium supported on alumina-magnesia was removed from the solution and dried to obtain palladium supported alumina-magnesia. The amount of palladium held was 1%.

Into 100 ml autoclave were charged methyl acetate (20.0 g), acetic acid (15.0 g), methyl iodide (13.0 g), 2,6-lutidine (0.363 g) and alumina-magnesia (7.2 g) as prepared above. The reaction was effected in the same way as in Example 1. Analysis showed 0.330 g of ethylidene diacetate, 0.116 g of acetic anhydride and 0.465 g of acetaldehyde. Atomic-absorption spectroscopy showed that, in comparison with palladium supported on the activated carbon before using, 99.97% of the palladium remained intact on the activated carbon.

Example 3

Into a 100 ml autoclave were charged methyl acetate (20.0 g), acetic acid (15.0 g), methyl iodide (13.0 g) and palladium (10%) supported on activated carbon (0.50 g) all being commercially available from Nippon Engelhard. Air was purged with nitrogen. Hydrogen under pressure was charged into the autoclave to 60 $Kg/cm^2$ Gauge and then carbon monoxide under pressure was charged into the autoclave to 180 $Kg/cm^2$ Gauge. The temperature in the autoclave was raised to 175°C. The reaction was continued for 4 hours with stirring at that temperature. After cooling the reaction mixture. Analysis showed that ethylidene diacetate and acetic anhydride were present therein.

Example 4

Into a 100 ml autoclave were charged acetic acid (20.0 g), methyl iodide (13.0 g), 3,6-lutidine (0.368 g) and palladium (5%) supported on activated carbon (1.43 g) as in Example 1. Air was purged with nitrogen. Dimethyl ether (20.0 g) was charged into the autoclave. After the temperature in the autoclave reached 175°C, mixed gas of CO and $H_2$ (ratio by volume of $CO:H_2$ of 4:1) was introduced into the autoclave until the pressure in the autoclave reached 200 $Kg/cm^2$ Gauge. The reaction was continued for 8 hours at 175°C with stirring. Analysis showed that the reaction mixture contained 0.73 g of ethylidene diacetate.

Control test 1

Into a 100 ml autoclave were charged methyl acetate (20.0 g), acetic acid (15.0 g), methyl iodide (8.0 g), 2,6-lutidine (0.325 g) and palladium·black (0.0647 g) being commercially available from

Nippon Engelhard. Thereafter, the procedure of Example 1 was repeated. Analysis showed that no ethylidene diacetate was formed.

Control test 2

Into a 100 ml autoclave were charged methyl acetate (20.0 g), acetic acid (15.0 g), methyl iodide (13.0 g), 2,6-lutidine (0.363 g) and rhodium (5%) supported on activated carbon in the form of powder (1.44 g) being commercially available from Nippon Engelhard. The reaction was continued at 175°C for 1.5 hours in the same way as in Example 1. Analysis showed that the reaction mixture contained 8.77 g of ethylidene diacetate, 3.93 g of acetic anhydride and 0.165 g of ethyl acetate. Rhodium supported on activated carbon was removed from the reaction mixture. Atomic-absorption spectroscopy showed that, in comparison with rhodium supported on the activated carbon before using, 37.4% of the rhodium remained intact on the activated carbon.

Control test 3

Into a 100 ml autoclave were charged methyl acetate (20.0 g), acetic acid (15.0 g), methyl iodide (13.0 g) and ruthenium (5%) supported on activated carbon in the form of powder (1.44 g) being commercially available from Nippon Engelhard. Thereafter, the procedure of Example 1 was repeated. Analysis showed that the reaction mixture contained ethylidene diacetate with acetic anhydride (0.0193 g), acetaldehyde (0.006 g) and ethyl acetate (0.131 g).

Examples 5—17

The procedures were repeated in accordance with Example 1. The raw materials employed, the reaction conditions and the results are shown in Table.

TABLE

| Ex. No. | Methyl acetate (g) | solvent (g) | main component (g) | methyl iodide (secondary component) (g) | promoter (g) | reaction conditions Temp. (°C) | Pressure (Kg/cm²) | time (Hr) |
|---|---|---|---|---|---|---|---|---|
| 5 | 20 | acetic acid 15 | 10% Pd/C 0.720 | 13 | triphenyl phosphine 0.887 | 175 | 100 | 4 |
| 6 | 20 | ditto 15 | 10% Pc/C 0.720 | 13 | tri-n-butyl amine 0.627 | 175 | 100 | 4 |
| 7 | 20 | ditto 15 | 5% Pd/C 1.44 | 13 | 4-vinyl-pyridine 0.356 | 175 | 100 | 4 |
| 8 | 20 | ditto 15 | 5% Pd/C 1.44 | 8 | ammonium acetate 0.522 | 175 | 100 | 8 |
| 9 | 20 | ditto 15 | 5% Pd/C 1.29 | 8 | pyridine 0.240 | 175 | 100 | 4 |
| 10 | 20 | ditto 15 | 5% Pd/C 1.44 | 8 | 2,6-lutidine 0.363 | 175 | 100 | 4 |
| 11 | 20 | ditto 15 | 5% Pd/C 1.29 | 8 | ditto 0.650 | 175 | 100 | 4 |
| 12 | 20 | ditto 15 | 5% Pd/C 1.17 | 4 | ditto 0.295 | 175 | 100 | 4 |
| 13 | 20 | ditto 15 | 5% Pd/C 1.29 | 8 | ditto 0.324 | 190 | 100 | 4 |
| 14 | 20 | ditto 15 | 5% Pd/C 1.29 | 8 | ditto 0.324 | 160 | 100 | 4 |
| 15 | 35 | none | 10% Pd/C 0.72 | 13 | ditto 0.363 | 175 | 100 | 4 |
| 16 | 20 | acetic acid 8.3 + acetic anhydride 6.7 | 5% Pd/C 1.29 | 8 | ditto 0.324 | 175 | 100 | 4 |
| 17 | 20 | acetic acid 15 | 5% Pd/C 5.16 | 8 | ditto 1.30 | 175 | 100 | 4 |

TABLE (Continued)

| Ex. No. | $CO:H_2$ by-volume | product ethylidene diacetate (g) | acetic anhydride (g) | acetaldehyde (g) | ethyl acetate (g) | proportion of Pd remained intact on carbon * (%) |
|---|---|---|---|---|---|---|
| 5 | 2:1 | 2.39 | 1.65 | 0.285 | 0.054 | 99.15 |
| 6 | 2:1 | 0.547 | 1.02 | 0.140 | 0.023 | 97.30 |
| 7 | 2:1 | 2.39 | 0.068 | 1.77 | 0.059 | 99.01 |
| 8 | 2:1 | 0.0584 | 0.063 | 0.015 | 0.034 | 97.90 |
| 9 | 2:1 | 1.68 | 0.076 | 1.37 | 0.051 | 99.98 |
| 10 | 3.8:1 | 2.41 | 0.515 | 0.732 | 0.083 | 99.98 |
| 11 | 2:1 | 3.24 | 0.413 | 0.590 | 0.055 | 99.98 |
| 12 | 2:1 | 1.14 | 1.03 | 0.469 | 0.044 | 99.97 |
| 13 | 2:1 | 2.42 | 1.44 | 0.468 | 0.071 | 99.99 |
| 14 | 2:1 | 0.788 | 0.238 | 0.477 | 0.029 | 99.98 |
| 15 | 2:1 | 0.553 | 5.20 | 0.198 | 0.183 | 99.99 |
| 16 | 2:1 | 4.26 | 2.65 | 0.305 | 0.136 | 99.99 |
| 17 | 2:1 | 4.72 | 0.226 | 3.38 | 0.146 | 99.91 |

* The proportion of Pd was based on Pd supported on activated carbon.

Example 18

Activated carbon (9.3 g) commercially available from Takeda Chemical Industries Inc. as Shirasagi for Brewage was heated to 80°C with 120 ml of water. Thereafter, palladium chloride (0.82 g) dissolved in concentrated hydrochloric acid (2 ml) and water (5 ml) was added to the suspension. A 37% formalin solution (1 ml) was added to the suspension. A 30% sodium hydroxide solution was added to the suspension until litmus paper showed the suspension to be weakly alkaline. The suspension was heated for 5 minutes with stirring. Palladium-supported on carbon was filtered and washed with water. Poly-4-vinylpyridine (molecular weight 97000) (2.47 g) was dissolved in methanol (50 ml). Water was added to the mixture until the total amount came to 100 ml. The palladium supported on activated carbon powder as prepared above was added to the mixture. Water and methanol were distilled under reduced pressure in a rotary evaporator. The resulting powder was vacuum dried.

Into a 100 ml autoclave were charged methyl acetate (20.0 g), acetic acid (15.0 g), methyl iodide (13.0 g) and palladium and poly-4-vinylpyridine supported activated carbon powder (1.61 g). The procedure of Example 1 was repeated. Analysis showed that the reaction mixture contained ethyldene diacetate (0.588 g), acetic anhydride (0.100 g) and acetaldehyde (0.809 g). Analysis showed that, in comparison with palladium supported on the activated carbon before using, 99.93% of the palladium remained intact on the activated carbon.

Example 19

Into a 100 ml autoclave were charged methyl acetate (20.0 g), acetic acid (15.0 g), methyl iodide (13.0 g), 2,6-lutidine (0.367 g) and palladium (5%) supported activated carbon powder (1.44 g) being commercially available from Nippon Engelhard. Thereafter, the procedure of Example 1 was repeated. Then the palladium-carbon was filtered. Analysis showed that the reaction mixture contained 4.12 g of ethylidene diacetate.

Using the palladium-carbon so filtered, the above procedure was repeated. As a result 4.22 g of ethylidene diacetate was obtained.

Example 20

Into an autoclave equipped with blade for agitation and filter were charged 2.88 g of palladium (5%) supported on activated carbon powder as in Example 1, 20.0 g of methyl acetate, 13.0 g of methyl iodide, 15.0 g of acetic acid and 0.734 of 2,6-lutidine. Air in the autoclave was purged with nitrogen. The nitrogen gas was purged with mixed gas of carbon monoxide and hydrogen (ratio by volume of $CO:H_2$ of 2:1).

The temperature in the autoclave was raised to 175°C. The above mixture of CO and $H_2$ under pressure was charged into the autoclave, until the pressure in the autoclave reached 100 $kg/cm^2$ Gauge. The reaction was continued for 3 hours at 175°C with stirring while maintaining the pressure in the autoclave at 100 $kg/cm^2$ Gauge by continuously introducing the mixed gas into the autoclave. A solution consisting of 41.0% of methyl acetate, 30.8% of acetic acid, 26.7% of methyl iodide and 1.5% of 2,6-lutidine was purged into the autoclaved continuously at a rate of 12.2 g/hour, and at the same time the resulting reaction mixture was continuously removed through a filter from the autoclave at 13.1 g/hour. During the reaction, the mixed gas was continuously introduced into the autoclave so as to maintain the pressure in the autoclave at 100 $Kg/cm^2$, and the temperature in the autoclave was maintained at 175°C. After 3 hours from starting of the reaction, the reaction mixture from the autoclave contained 11.0% of ethylidene diacetate. After 10 hours, the reaction mixture contained 10.7% of ethylidene diacetate. After 10 hours, the reaction was discontinued. The palladium supported on activated carbon was removed from the autoclave. Atomic-absorption spectroscopy showed that in comparison with palladium supported on the activated carbon before using, 99.93% of the palladium remained intact on the activated carbon.

**Claims**

1. A process for producing ethylidene diacetate and/or acetaldehyde which comprises hydrocarbonylating methyl acetate or dimethyl ether with carbon monoxide and hydrogen in the presence of a catalyst consisting essentially of (i) as a main component a group VIII noble metal and (ii) as a secondary component at least one halide selected from iodides, bromides and mixtures thereof under substantially anhydrous conditions characterised in that the main catalyst component comprises palladium physically supported on a porous inorganic material having a pore volume of 0.03 to 2.5 $cm^3/g$ and a surface area of 0.1 to 1500 $m^2/g$.

2. The process as defined in Claim 1 wherein the porous inorganic material for supporting palladium is selected from carbon, activated carbon, graphite, bone black, alumina, silica, silica alumina, barium sulfate, natural or synthetic zeolite, spinel, magnesia adhered alumina, thoria, titanium oxide, ziroconium oxide, thorium oxide, lanthanum oxide, cerium oxide, zinc oxide, tantalum, clay, diatomaceous earth, asbestos, pumice stone, bauxite, terra alba, natural and treated terra alba, silicon carbide, zeolite molecular sieves, ceramic honeycomb, boria, cements, alundum, corundum, brick, talc, and gypsum.

3. The process as defined in Claim 2 wherein the porous inorganic material for supporting palladium is selected from activated carbon, carbon, graphite, bone black, alumina, silica, silica alumina and barium sulfate.

4. The process as defined in Claim 1 wherein the secondary component is at least one iodide.

5. The process as defined in Claim 1 wherein the amount of the main component employed is in the range of $1 \times 10^{-4}$ to 25 percent by weight on the basis of weight of a reaction solution in terms of metal.

6. The process as defined in Claim 1 wherein the amount of the secondary component employed is in the range of $1 \times 10^{-6}$ to 15 mol per 1 litre of a reaction solution in terms of halogen atom.

7. The process as defined in Claim 1 wherein the reaction is effected in the presence of a promoter selected from ammonia, ammonium salts and organic compounds containing nitrogen or phosphorus.

8. The process as defined in Claim 7 wherein the promoter is selected from the group consisting of organic compounds containing trivalent phosphorous atom or trivalent nitrogen atom.

9. A process as defined in any preceding claim wherein the main catalyst component is prepared by depositing palladium on the porous inorganic material by impregnation, blending, adsorption, coprecipitation or ion-exchange.

10. A process as defined in any preceding claim wherein the average semi-diameter of the pores in the porous inorganic material is in the range of from 0.1 to 2000A.

11. A process as defined in any preceding claim wherein the proportion by weight of palladium supported on the porous inorganic material is in the range from 0.01 to 30%.

**Revendications**

1. Procédé de préparation de diacétate d'éthylidène et/ou d'acétaldéhyde, qui comprend une hydrocarbonylation d'acétate de méthyle ou d'éther méthylique par l'oxyde de carbone et l'hydrogène en présence d'un catalyseur consistant essentiellement (i) en un constituant principal constitué par un

métal noble du groupe VIII et (ii) en un constituant secondaire constitué par au moins un halogène choisi parmi les iodures, les bromures et des mélanges de ceux-ci, dans des conditions sensiblement anhydres, caractérisé en ce que le constituant principal du catalyseur comprend du palladium matériellement supporté par une matière inorganique poreuse à volume des pores de 0,03 à 2,5 $cm^3/g$ et une superficie de 0,1 à 1500 $m^2/g$.

2. Procédé suivant la revendication 1, dans lequel la matière inorganique poreuse de support du palladium est choisie parmi le carbone, le carbone activé, le graphite, le noir d'os, l'alumine, la silice, l'aluminosilicate, le sulfate de baryum, une zéolithe naturelle ou synthétique, le spinelle, l'alumine adhérée par magnésie, le bioxyde de thorium, l'oxyde de titane, l'oxyde de zirconium, l'oxyde de thorium, l'oxyde de lanthane, l'oxyde de cérium, l'oxyde de zinc, le tantale, l'argile, la diatomite, l'amiante, la pierre ponce, la bauxite, la terra alba, la terra alba naturelle et traitée, le carbure de silicium, les tamis moléculaires zéolithiques, une matière alvéolaire céramique, l'oxyde de bore, les ciments, l'alundum, le corindon, la brique, le talc et le gypse.

3. Procédé suivant la revendication 2, dans lequel la matière inorganique poreuse supportant le palladium est choisi parmi le carbone activé, le carbone, le graphite, le noir d'os, l'alumine, la silice, l'aluminosilicate et le sulfate de baryum.

4. Procédé suivant la revendication 1, dans lequel le constituant secondaire est au moins un iodure.

5. Procédé suivant la revendication 1, dans lequel la quantité de constituant principal utilisée se trouve dans la palge de $1.10^{-4}$ à 25% en poids par ràpport au poids d'une solution réactionnelle, exprimé en métal.

6. Procédé suivant la revendication 1, dans lequel la quanntité de constituant secondaire utilisée se truve dans la plage de $1.10^{-6}$ à 15 moles par litre d'une solution réactionnelle, exprimé en atomes d'halogène.

7. Procédé suivant la revendication 1, dans lequel la réaction est effectuée en présence d'un promoteur choisi parmi l'ammoniac, les sels ammoniacaux, les composés organiques contenant de l'azote ou du phosphore.

8. Procédé suivant la revendication 7, dans lequel le promoteur est choisi dans le groupe comprenant les composés organiques contenant un atome de phosphore trivalent et un atome d'azote trivalent.

9. Procédé suivant l'une quelconque des revendications précédentes, dans lequel le constituant principal du catalyseur est préparé en déposant du palladium sur la matière inorganique poreuse par imprégnation, adsorption, co-précipitation ou échange d'ions.

10. Procédé suivant l'une quelconque des revendications précédentes, dans lequel le demi-diamètre moyen des pores de la matière inorganique poreuse se trouve dans la plage de 0,1 à 2000 A.

11. Procédé suivant l'une quelconque des revendications précédentes, dans lequel la proportion en poids du palladium supporté par la matière inorganique poreuse se trouve dans la plage de 0,01 à 30%.

## Patentansprüche

1. Verfahren zur Herstellung von Äthylidendiacetat und/oder Acetaldehyd, wobei Methylacetat oder Dimethyläther mit Kohlenstoffmonoxyd und Wasserstoff unter im wesentlichen wasserfreien Bedingungen mit einem Katalysator hydrocarbonyliert wird, der im wesentlichen aus (i) einem Edelmetall der Gruppe VIII als Hauptkomponente und (ii) wenigstens einem Halogenid als sekundärer Komponente besteht, das aus Jodiden, Bromiden und Gemischen davon ausgewählt wird, dadurch gekennzeichnet, daß die Katalysatorhauptkomponente Palladium umfaßt, das auf einem porösen anorganischen Material vorliegt, das ein Porenvolumen von 0,03 bis 2,5 $cm^3/g$ und eine spezifische Oberfläche von 0,1 bis 1500 $m^2/g$ aufweist.

2. Verfahren nach Anspruch 1, wobei das poröse anorganische Material, auf dem das Palladium vorliegt, ausgewählt wird aus Kohlenstoff, Aktivkohle, Graphit, Knochenkohle, Aluminiumoxid, Siliciumoxid, Aluminiumsilicat, Bariumsulfat, natürlichem oder synthetischen Zeolit, Spinel, an Aluminiumoxid haftenden Magnesiumoxid, Thorerde, Titanoxid, Zirkoniumoxid, Thoriumoxid, Lanthanoxid, Ceroxid, Zinkoxid, Tantal, Ton, Diatomeenerde, Asbest, Bimsstein, Bauxit, Terra Alba, natürliche und behandelte Terra Alba, Siliciumcarbid, Zeolit-Molekularsiebe, Keramikzellen, Boroxid, Zement, Alaun, Korund, Ziegelstein, Kalk und Gips.

3. Verfahren nach Anspruch 2, wobei das poröse anorganische Material, auf dem das Palladium vorliegt, ausgewählt wird aus Aktivkohle, Kohlenstoff, Graphit, Knochenkohle, Aluminiumoxid, Siliciumoxid, Aluminiumsilicat und Barium-sulfat.

4. Verfahren nach Anspruch 1, wobei die sekundäre Komponente wenigstens ein Jodid ist.

5. Verfahren nach Anspruch 1, wobei die Menge der verwendeten Hauptkomponente im Bereich zwischen $1 \times 10^{-4}$ und 25 Gewichtsprozent auf der basis des Gewichts der Reaktionslösung, bezogen auf das Metall, liegt.

6. Verfahren nach Anspruch 1, wobei die Menge der verwendeten sekundären Komponente im Bereich zwischen $1 \times 10^{-6}$ bis 15 Mol je Liter Reaktionslösung, bezogen auf Halogenatom, liegt.

7. Verfahren nach Anspruch 1, wobei die Reaktion in Gegenwart eines Promotors durchgeführt wird, der aus Ammoniak, Ammoniumsalzen sowie organischen Verbindungen, die Stickstoff oder Phosphor enthalten, ausgewählt wird.

8. Verfahren nach Anspruch 7, wobei der Promoter aus einer Gruppe ausgewählt wird, die aus organischen Verbindungen besteht, die ein dreiwertiges Phosphoratom oder dreiwertiges Stickstoffatom enthalten.

9. Verfahren nach einem der vorstehenden Ansprüche, wobei die Katalysatorhauptkomponente hergestellt wird, indem Palladium auf einem porösen anorganischen Material durch Imprägnieren, Mischen, Absorption, Copräzipitation oder Ionenaustausch abgelagert wird.

10. Verfahren nach einem der vorstehenden Ansprüche, wobei der mittlere Halbdurchmesser der Poren des porösen organischen Materials im bereich zwischen 0,1 bis 2000 Å liegt.

11. Verfahren nach einem der vorstehenden Ansprüche, wobei der Gewichtsanteil des Palladiums, das auf dem porösen anorganischen Material vorliegt, im bereich zwischen 0,1 und 30% liegt.